# EUROPEAN PATENT SPECIFICATION

(11) **EP 0 391 503 B1**
(45) Date of publication and mention of the grant of the patent: **15.06.1994**
(21) Application number: 90201279.8
(22) Date of filing: 05.02.1988
(51) Int. Cl.: C12P 33/06, C07J 7/00

(54) **Preparation of 7 beta-hydroxy-4-pregnene-3,20-dione derivatives**
Herstellung von 7-beta-Hydroxy-4-pregnen-3,20-dion-Derivaten
Préparation de dérivés de 7-bêta-hydroxy-4-pregnène-3,20-dione

(30) Priority: 06.02.1987 JP 24597/87; 15.06.1987 JP 147017/87
(43) Date of publication of application: 10.10.1990
(62) Divisional of application: 88901463.5
(73) Proprietor: SNOW BRAND MILK PRODUCTS CO. LTD., Sapporo-shi Hokkaido (JP)
(72) Inventor: Yoshihama, Makoto, Utsunomiya-shi, Tochigi, 321-01 (JP); Miyata, Nobuo, Utsunomiya-shi, Tochigi, 321 (JP); Tamura, Koji, Yonoshi, Saitama 338 (JP); Nakakoshi, Masamichi, Utsunomiya-shi, Tochigi 321-01 (JP)
(74) Representative: Ford, Michael Frederick

(56) References cited:
- CHEMICAL ABSTRACTS, vol. 98, no. 7, 14th February 1983, page 514, abstract no. 51853m, Columbus, Ohio, US; A. MUKHERJEE et al.: "Metabolism of progesterone by Aspergillus fumigatus", & J. STEROID BIOCHEM. 1982, 17(4), 443-6
- CHEMICAL ABSTRACTS, vol. 97, no. 9, 30th August 1982, page 323, abstract no. 69011z, Columbus, Ohio, US; A. MUKHERJEE et al.: "Metabolism of progesterone by an Aspergillus species", & INT. CONF. CHEM. BIOTECHNOL. BIOL. ACT. NAT. PROD., [PROC.], 1ST 1981, 3(2), 155-7
- CHEMICAL ABSTRACTS, vol. 55, no. 17, 21st August 1961, column no. 16903 f-i, Columbus, Ohio, US; K. TSUDA et al.: "Microbiological hydroxylation of steroids. X. 7beta,15beta-dihydroxypregn-4-ene-3,20-dione", & CHEM. PHARM. BULL. (TOKYO) 8, 626-8(1960)
- JOURNAL OF CHEMICAL ECOLOGY, vol. 13, no. 1, January 1987, pages 35-38, Plenum Publishing Corp., New York, US; J. MEINWALD et al.: "Defensive steroids from a carrion beetle"

## Description

This invention relates to the preparation of pregnene derivatives used as intermediates for the synthesis of progesterone derivatives having ovulation-inhibiting activity. A group of these compounds is expected to be used in the field of medicine. Cell differentiation activity is also found on a part of these compounds and hence utilisation as drugs is also expected in this area.

This application is a divisonal application out of our European application (EP-A-301102) which is referred to herein as the parent application.

7β-hydroxy-4-pregnene-3,20-dione derivatives have been prepared by various methods. J. STEROID BIOCHEM. 1982 17(4) pages 443-6 (Mukherjee et al) discloses isolation of the 15β hydroxy derivative following metabolism of progesterone by A. fumigatus. Also, INT. CONF. CHEM. BIOTECHNOL. BIOL. ACT. NAT. PROD (PROC.) 1ST 1981, 3(2), pages 155-7 (Mukherjee et al) describes metabolism of progesterone by an Aspergillus strain to provide the 15β hydroxy derivative. In addition CHEM. PHARM. BULL (TOKYO) 8, pages 626-8 (1960) (K Tsuda et al) also concerns a method for preparing the 15β derivative by microbiological hydroxylation of progesterone by Diplodia tubericola.

7β-hydroxy-4-pregnene-3,15,20-trione may also be produced by oxidation of oxygenated pregnenes isolated from Sipha novaboracensis, as described in JOURNAL OF CHEMICAL ECOLOGY, vol 13, No. 1, January 1987, pages 35-38 (J. Meinwald et al).

The present inventors have researched the action of microorganisms belonging to Acremonium sp., e.g. a strain of mold fungi, in a substrate of 4-pregnene-3,20-dione. As a result, it has been found that two certain pregnene derivatives are produced. According to the present invention there is provided a method for preparing a 7β-hydroxy-4-pregnene-3,20-dione derivative represented by the formula (I): 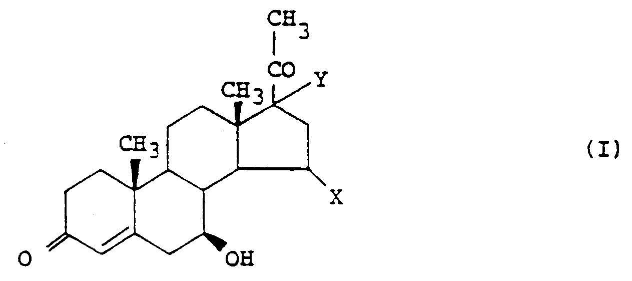
wherein X is =0 or αHβOH, and Y is αH which is characterized in that a microorganism belonging to Acremonium sp., is brought into contact with 4-pregnene-3,20-dione.

The present inventors have further found that esters of the pregnene derivatives can be obtained.

The pregnene derivatives can be used as intermediates for the manufacture of progesterone derivatives. These pregnene derivatives including the above mentioned esters exhibit also cell differentiation activity.

One particular pregnene derivative produced by the method of this invention has the chemical name 7β-Hydroxy-4-pregnene-3,15,20-trione.

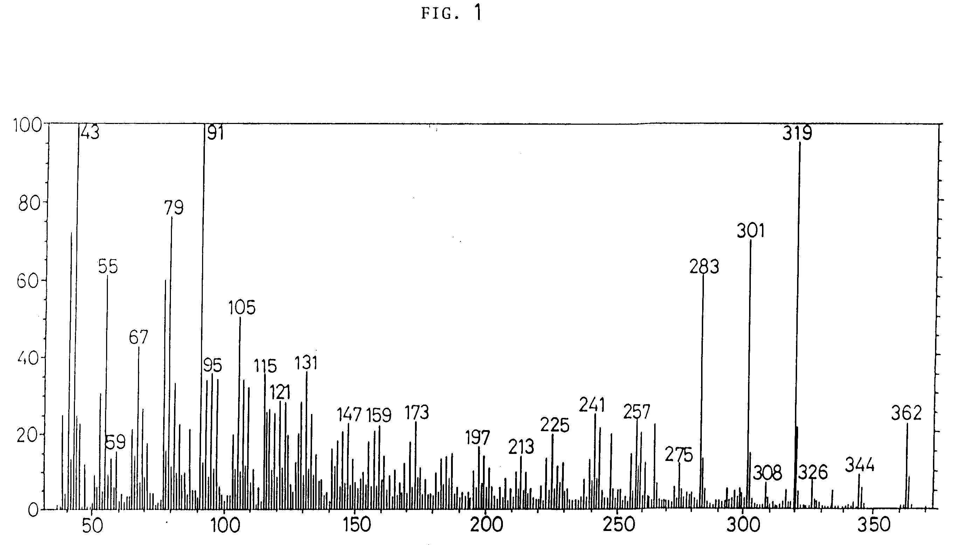

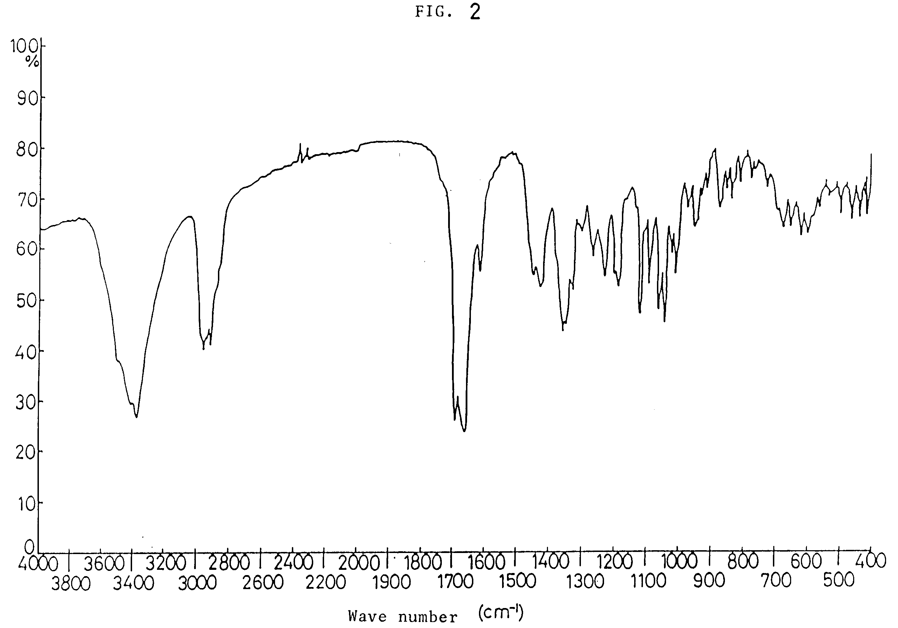

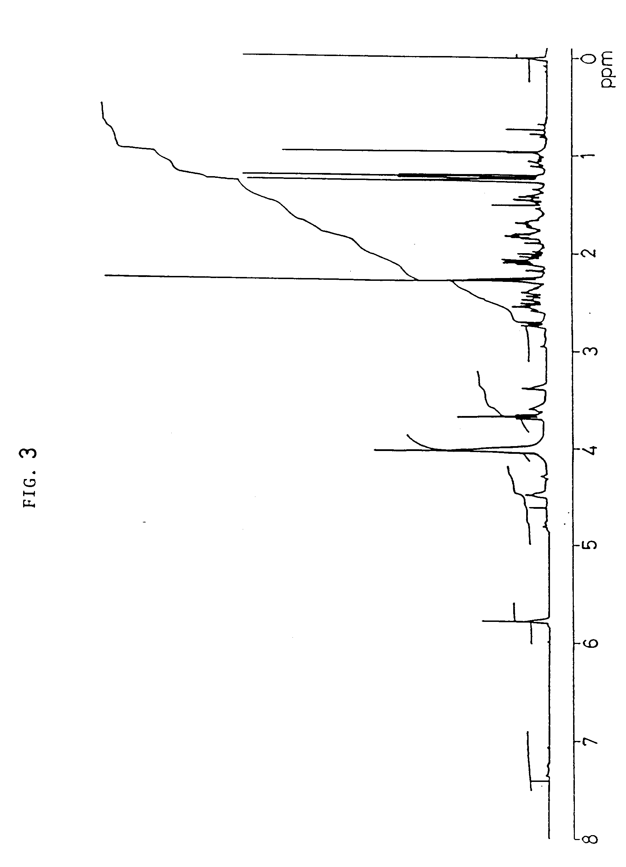

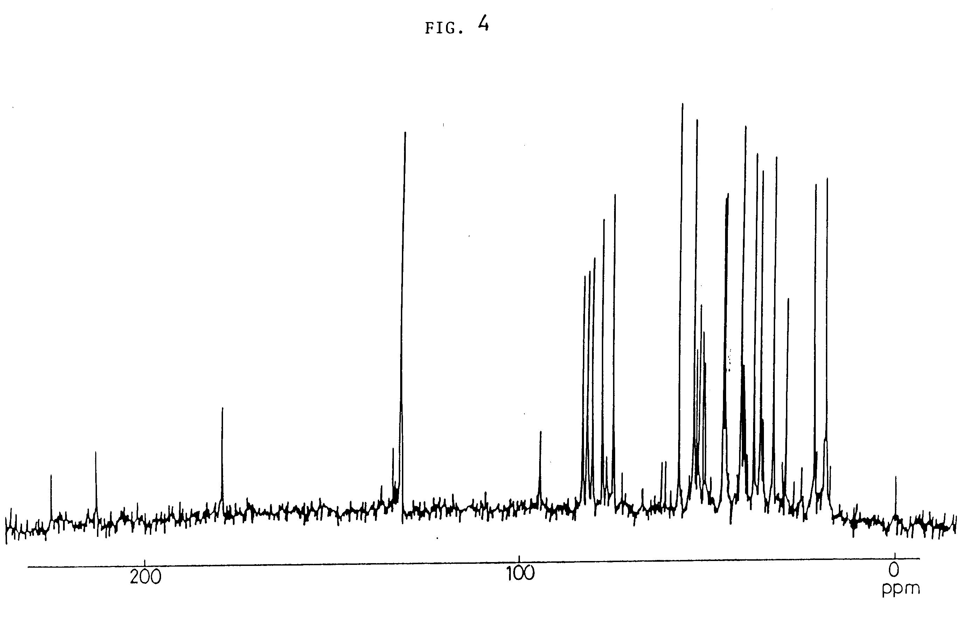

Figures 1-4 relate to one compound produced by the microorganism, namely 7β, 15β 17α-Trihydroxy-4-pregnene-3,20-dione. Figure 1 illustrates an EI mass spectrum. Figure 2 shows an infrared absorption spectrum. Figure 3 shows a proton nuclear magnetic resonance spectrum. Figure 4 illustrates a ¹³C-nuclear magnetic resonance spectrum.

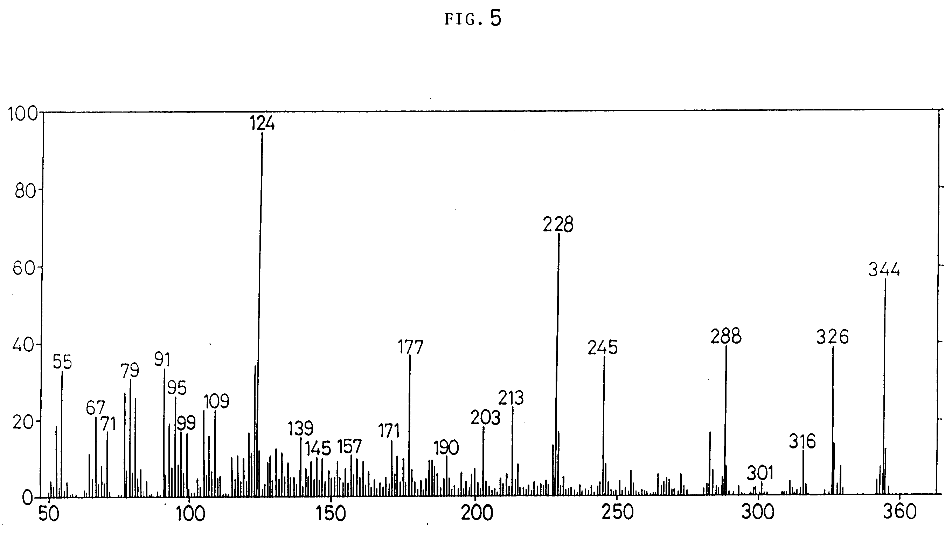

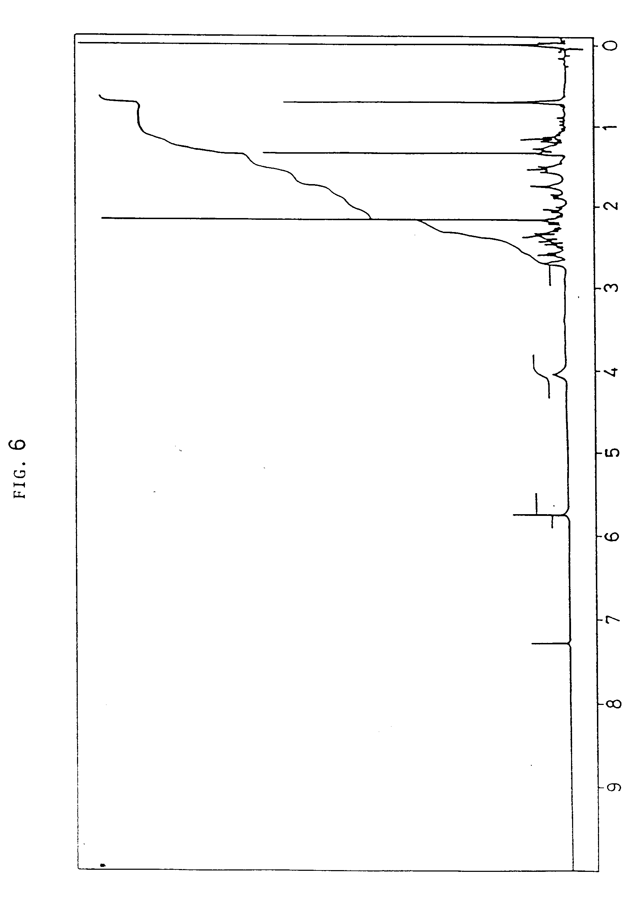

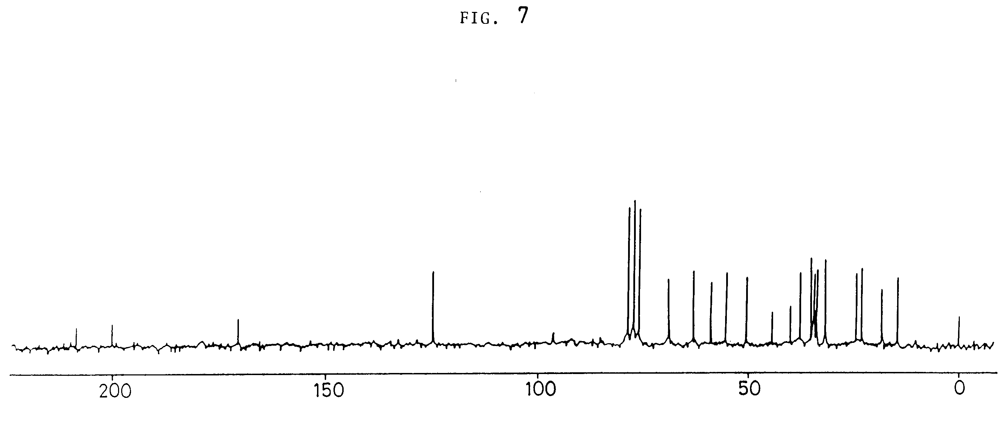

Figures 5-7 relate to 7β-hydroxy-4-pregnene-3,15,20 trione, which is a compound produced by the invention. Figure 5 illustrates an EI mass spectrum. Figure 6 illustrates a proton nuclear magnetic resonance spectrum. Figure 7 shows a ¹³C-nuclear magnetic resonance spectrum.

This invention is to provide a pregnene derivative which is used as an intermediate for the manufacture of progesterone derivatives having progestin activities such as ovulation-inhibiting activity and also has itself cell differentiation activity. This invention will hereinafter be illustrated in detail by way of examples.

### Culture of microorganisms and conversion of substrate by the microorganisms

Acremonium strictum NN106 preserved in Hungarian National Institute of Hygiene (FERM P-9143), which is a strain of mold fungi was cultured with shaking in a medium containing carbon sources such as malt extract, peptone, nitrogen sources such as soybean meal, and inorganic salts. In carrying out the culture, the strain was inoculated on 100 ml of a culture medium having, for example, a below described composition in an Erlenmeyer flask of 500 ml volume. The culture was incubated and shaken simultaneously in a incubator with a rotary shaker at a rate of 200 rpm for 48 hours at 24°C.

| | |
|---|---|
| Malt extract | 30 g |
| Peptone | 20 g |
| Soybean meal | 10 g |
| Potassium phosphate, monobasic | 5 g |
| Magnesium sulfate | 5 g |
| Purified water | 1000 ml |

The above strain was deposited as FERM P-9143 in Fermentation Research Institute of the Agency of Industrial Science and Technology on January 21, 1987.

4-Pregnene-3,20-dione as a substrate was dissolved in dimethylformamide in advance so as to obtain a substrate concentration of 0.1 g/ml.

To the culture solution obtained above, 2 ml of the above substrate solution was added and a reaction was carried out for 24- 48 hours under the same conditions as above.

After completing the reaction, solid matters and cells were removed from the resultant culture solution by centrifugal separation. The resultant supernatant was extracted three times, each time using one third of its volume of ethyl acetate. The solvent was removed from the extracted solution with a rotary evaporator.

A crude fraction thus obtained was dissolved in a small amount of chloroform (or methanol) and passed through a silica gel column HPLC (20 mm diameter x 300 mm length). The adsorbed fractions were eluted with a solvent mixture (chloroform:methanol = 98:2) and fractionated.

The eluate could be divided into at least two fractions. A constituent contained in one of the fractions was identified as 7β,15β,17α-trihydroxy-4-pregnene-3,20-dione (a compound of our parent application) by the below described physicochemical properties obtained as a result of structural analysis. The yield was 9.6 mg.

A constituent contained in an other fraction which was eluted earlier than above, for example, at 6 minutes as compared to 26 minutes for the above compound was identified as 7β-hydroxy-4-pregnene-3,15,20-trione (a compound made by the process of this invention) by the same method as above.

7β,15β,17α-Trihydroxy-4-pregnene-3,20-dione is represented by the chemical formula (II): 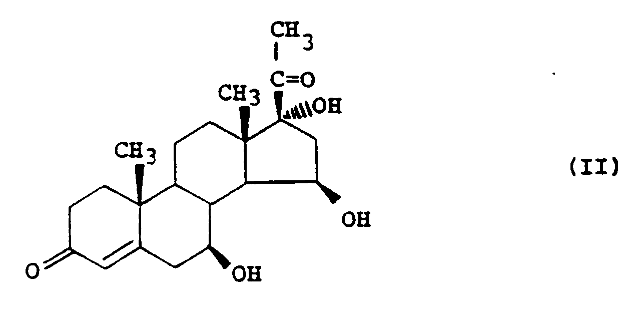
The compound can be identified by the following physicochemical properties.

| | |
|---|---|
| (1) Appearance | White powder |
| (2) Molecular weight | 362 |
| (3) Molecular formula | C₂₁H₃₀O₅ |
| (4) Melting point | 248-250°C |
| (5) Specific rotation | [α]D= +41.4° |
| (6) EI mass spectrum | m/Z = 362. See Figure 1. |
| (7) Infrared absorption spectrum | See Figure 2. |
| (8) Proton nuclear magnetic resonance spectrum | See Figure 3. |
| (9) ¹³C-nuclear magnetic resonance spectrum | See Figure 4. |

7β-Hydroxy-4-pregnene-3,15,20-trione is represented by the chemical formula (III): 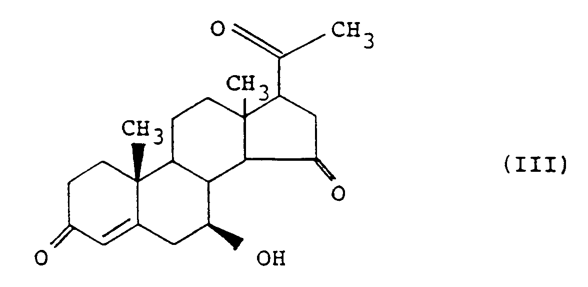
The compound can be identified by the following physicochemical properties.

| | |
|---|---|
| (1) Appearance | White powder |
| (2) Molecular weight | 344 |
| (3) Molecular formula | C₂₁H₂₈O₄ |
| (4) Melting point | 254-256°C |
| (5) EI mass spectrum | m/Z=344, See Figure 5. |
| (6) Proton nuclear magnetic spectrum | See Figure 6. |
| (7) ¹³C-nuclear magnetic resonance spectrum | See Figure 7. |

Pregnene derivatives can be esterified. For example the compound 7β,15β,17α-Trihydroxy-4-pregnene,3,20-dione can be used as an intermediate for the synthesis of progesterone derivatives having ovulation-inhibiting activity. It can be converted to, 7β,15β-diacetyl-17α-hydroxy-4-pregnene-3,20-dione by reaction with acetic anhydride in pyridine to give the above acetylated derivative in the yield of 96%.

In accordance with the above mentioned procedures, disuccinic acid ester derivative or diglutaric acid ester is obtained respectively by reacting succinic anhydride or glutaric anhydride in place of acetic anhydride.

Progesterone and the below described compound, which is obtainable by the present method, were respectively allowed to act for 48 hours on myelogenic leukemia M1 cells derived from SL mouse.

The cell differentiation activity was measured by observing morphological change of the cells under a microscope. The results are indicated in the Table below

**Table**

| Cell differentiation activity on M1 cells | |
|---|---|
| Progesterone | - |
| 7β-Hydroxy-4-pregnene-3,15,20-trione | 50% |

Reference to microorganisms deposited pursuant to Regulation 13 bis.
- Deposit Organization:: Fermentation Research Institute of the Agency of Industrial Science and Technology
- Address:: 1-3, Higashi-1-chome, Tsukuba-shi, Ibaraki-ken, JAPAN
- Deposit No.:: FERM P-9143
- Date of Deposit:: January 21, 1987

## Claims

1. A method for preparing a 7β-hydroxy-4-pregnene-3,20-dione derivative represented by the formula (I): 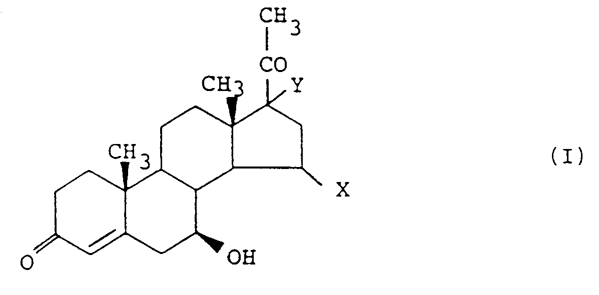 wherein X is =0 or αHβOH, and Y is αH which is characterized in that a microoorganism belonging to Acremonium sp., is brought into contact with 4-pregnene-3,20-dione.

2. A method according to claim 1 wherein X = 0 so that the compound of formula (I) is 7β-hydroxy-4-pregnene-3,15,20-trione.

## Patentansprüche

1. Verfahren zur Herstellung eines Derivates von 7β-Hydroxy-4-pregnen-3,20-dion, dargestellt durch die Formel (I): 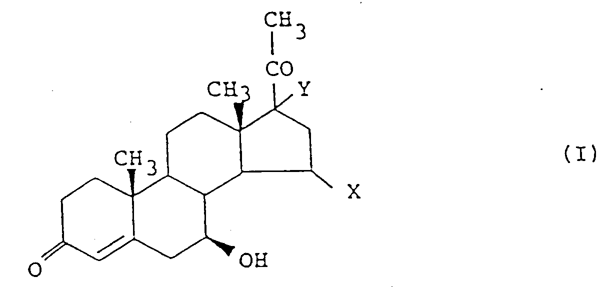 wobei X = O oder αHβOH und Y = αH, dadurch gekennzeichnet, daß ein Mikroorganismus der Spezies Acremonium mit 4-Pregnen-3,20-dion in Berührung gebracht wird.

2. Das Verfahren nach Anspruch 1, wobei X = O, so daß die Verbindung der Formel (I) 7β-Hydroxy-4-pregnen-3,15,20-trion ist.

## Revendications

1. Procédé pour préparer un dérivé 7β-hydroxy-4-prégnène-3,20-dione représenté par la formule (I): 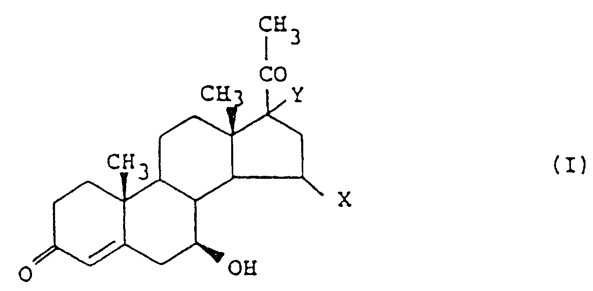 dans laquelle X représente un atome O ou un groupement αHβOH, et Y représente un atome αH, qui est caractérisé en ce qu'un microorganisme appartenant à Acremonium sp. est mis en contact avec la 4-prégnène-3,20-dione.

2. Procédé selon la revendication 1, dans lequel X représente un atome O de sorte que le composé de formule (I) est la 7β-hydroxy-4-prégnène-3,15,20-trione
